# EUROPEAN PATENT APPLICATION

(11) **EP 1 457 177 A1**
(43) Date of publication of application: **15.09.2004**
(21) Application number: 03251570.2
(22) Date of filing: 14.03.2003
(51) Int. Cl.: A61F 5/058

(54) **Bent-up splint and method of manufacturing the same**

(71) Applicant: Moon, Young Hoon, Busan City (KR); Moon, Byong Soon, Yangsan-City, Kyungnam (KR)
(72) Inventor: Moon, Young Hoon, Busan City (KR); Moon, Byong Soon, Yangsan-City, Kyungnam (KR)
(74) Representative: Jenkins, Peter David

(57) **Abstract**

The bent-up splint (100) for protecting/supporting the affected parts comprises an endoderm (2) and an integument (3) having an "U" shape to which two bent-up plates having a horizontal section (1a) and a vertical section (1b) are adhered by an adhering means, the endoderm (2) and integument (3) having a bent-up portion for allowing an angle to be formed at its central portion; and a heartwood (4) being a sandwiched fabric in which a plurality of split fabrics are formed between the two fabric surfaces (4a), wherein an outer circumference portion of the two bent-up plates having the horizontal section and the vertical section is adhered by an adhering means, so the cross section of the circumference portion is a "U" shape, wherein a bent-up portion has an angle at its central portion, the endoderm (2) and the integument (3) are applied with a solidification solution such as polyurethane or gypsum, the heartwood (4) is inserted between the endoderm (2) and an integument (3).

## Description

The invention relates generally to a bent-up splint used to wrap affected parts such as a fractured part and a ligament fractured part so that they do not move, and method of manufacturing the same. More particularly, the present invention relates to a bent-up splint that can facilitate a surgical operation by shaping the bent-up splint similar to an operation shape of a bent-up portion such as an elbow at a bent-up shape in which the wrinkles are not formed in the bent-up splint and that can increase an effect of a medical treatment by closely fixing the bent-up splint to the affected parts so that the splint does not float, and method of manufacturing the same.

Generally, a splint for use in orthopedic surgery is used to partially or entirely firmly wrap fractured joints, broken bones, extension of a ligament and fractured muscular organization so that they can be firmly support/fixed.

At this, time, it is required that materials used for the splint be flexible enough to shape the curved damaged parts and be adequate in solidification time or operation time. Further, it is required that the materials have a mechanical strength sufficiently enough to maintain its structure. A method that has been widely used as splint materials meeting these requirements, include a method of wrapping bandage on which gypsum material is applied to the affected parts. The gypsum bandage has been widely used as the bent-up splint since it can easily form the shape of the curved affected parts due to its flexible property after solidification, has adequate solidification time and operation time, and has a high mechanical strength. This gypsum bandage, however, requires mush time taken to wrap the wounded parts because it must be wrapped on the affected parts in a sufficient thickness.

In order to solve this problem, there has been proposed a material for a splint being textiles such as gypsum bandage, glass fiber or synthetic fiber textiles is immersed into polyurethane resin for wet solidification (1 liquid phase) and is then folded 8 ― 10 times. Thus, there is an advantage that this splint can reduce the use time. This splint is shaped with curve along the curved angle of humans' curved parts such as elbow, knee, heel, etc since it is textiles formed in a rectangular shape. Thus, there is a disadvantage that wrinkle portions are inevitably occurred at the curved parts. Further, the splint lacks in the shaping capability closely wrapping the affected parts depending on the curved shapes from the affected parts, for example, the thickness of the wrist and ankle, the thickness of the ankle and calf, etc. In this case, the splint get loose from the affected parts and thus does not firmly support the affected parts, thus adversely affecting curing of the operation portion such as broken parts.

In order to solve this problem, there has been proposed a splint made of a thermoplastic resin. The splint is molded to have the cross section of a 'U' shape and a 'L' shape and has an endoderm attached as a sponge. At this time, the splint is molded to be an 'L' shape. As the cross-sectional view of the splint is a 'U' shape, it must be made soft at the temperature of about 90°C in the oven in order for the splint to be closely adhered to the affected parts. The soft splint must be then attached to the affected parts along the curved parts of the affected parts by hands. During this operation, the soft splint of a thermoplastic resin becomes cool. Thus, the splint could not be completely attached to the affected parts once. Therefore, the splint must be made soft into the oven more than once. In addition, the splint must get cool for about 20 minutes after the shaping. Thus, there are problems that the operation is inconvenient and a lot of time is required.

The present invention is contrived to solve these problems and an aim of the present invention is therefore to provide a bent-up splint capable of facilitating an operation and increasing an effect of the operation, in such a way that the splint is shaped to be a bent-up state like an operation shape in which wrinkles are not formed and the splint can be thus adhered to the affected parts so that the splint does not get loose, and method of manufacturing the same.

In order to accomplish the above aim, a bent-up splint for protecting/supporting the affected parts according to the present invention, is characterized in that it comprises an endoderm and an integument having an 'U' shape to which two bent-up plates having a horizontal section and a vertical section are adhered by an adhering means, the endoderm and integument having a bent-up portion for allowing an angle to be formed at its central portion; and a heartwood being a sandwiched fabric in which a plurality of split fabrics are formed between the two fabric surfaces, wherein an outer circumference portion of the two bent-up plates having the horizontal section and the vertical section is adhered by an adhering means, so the cross section of the circumference portion is a 'U' shape, wherein a bent-up portion has an angle at its central portion, the endoderm and the integument are applied with a solidification solution such as polyurethane or gypsum, the heartwood is inserted between the endoderm and an integument.

The bent-up plate is processed within a mold along the shape of the splint by means of an ultrasonic wave, so that the angle of the horizontal section 1a and the vertical section 1b can be maintained. Therefore, the cut cross section is smooth, there occur no wrinkles and loose fabrics can be prevented.

The aforementioned aspects and other features of the present invention will be explained in the following description, taken in conjunction with the accompanying drawings, wherein:
Fig. 1 is a perspective view of a partially cut-away bent-up splint;
Fig. 2 is a longitudinal cross-section view of the bent-up splint in Fig. 1;
Fig. 3 is a cross-sectional view of the bent-up splint taken along line "B-B" in Fig. 1;
Fig. 4a ∼ Fig. 4d illustrate processes of manufacturing the bent-up splint according to the present invention;
Fig. 5a ∼ Fig. 5c illustrate heartwood built into the bent-up splint according to the present invention;
Fig. 6 shows another embodiment of a bent-up means;
Fig. 7 is an unfolded view of the bent-up means in Fig. 6;
Fig. 8 shows a still another embodiment of a bent-up means; and
Fig. 9 is an unfolded view of the bent-up means in Fig. 8.

The present invention will be described in detail by way of a preferred embodiment with reference to accompanying drawings.

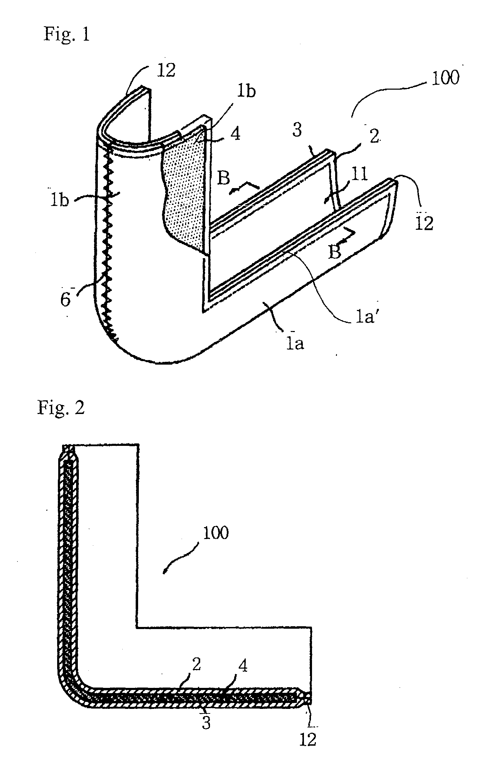

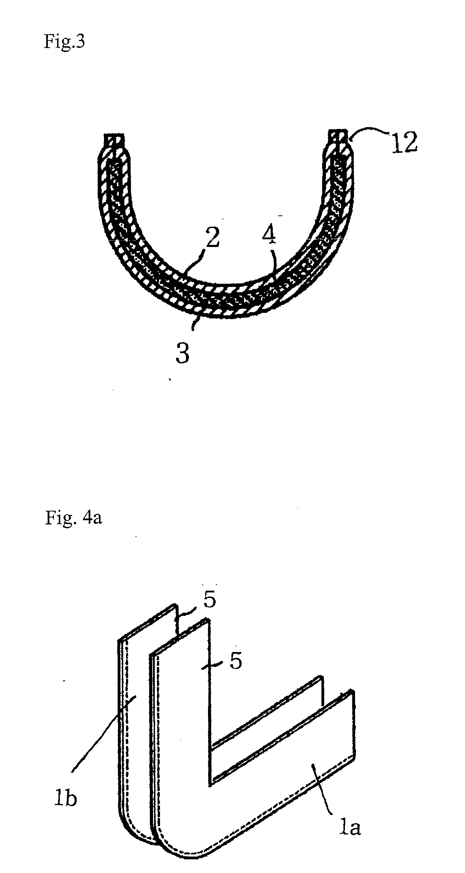

Referring now to Figs. 1, 2, 3 and 4, an endoderm 2, an integument 3 and heartwood 4 being bent-up plate constituting a bent-up splint 100 according to the present invention are bent-up means. The bent-up plates are cut to have a rectangular or given angle having horizontal sections 1a, 1a' and vertical sections 1b, 1b' by means of a molding cutter or an ultrasonic processing. The cut bent-up plates are the endoderm 2, integument 3 and heartwood 4 in which rectangular or given angle having the horizontal sections 1a, 1a' and the vertical sections 1b, 1b' similar to the operation shape. The heartwood 4 is intervened between the endoderm 2 and the integument 3 and is intervened by an ultrasonic wave thermal melting processing or seaming process. The endoderm 2 contacts the affected parts and is made of woven fabric, cotton or sponge. Also, a material for the integument 3 is exposed outside and is made of woven fabric or cotton having the durability. The endoderm 2 and the integument 3 each include two sheets of bent-up plates 5, 5', which are then seamed to an outer circumference portion 6 by an ultrasonic wave process or seaming process.

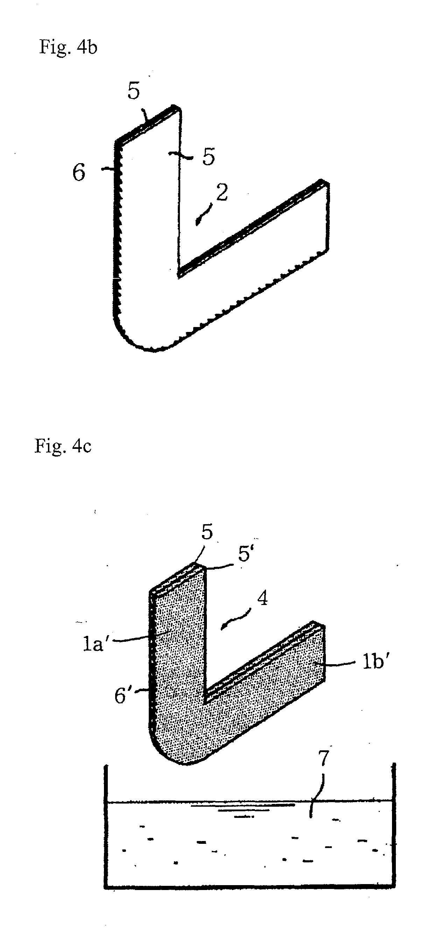

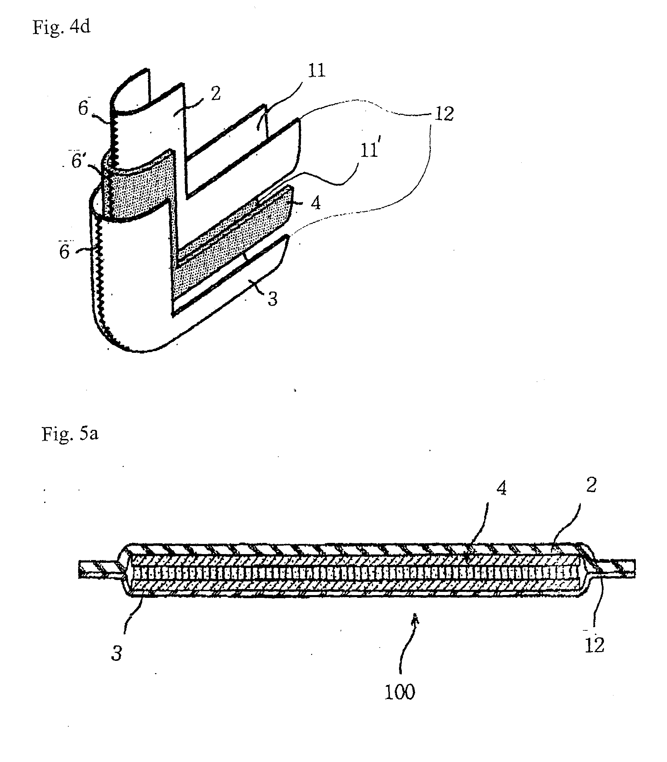

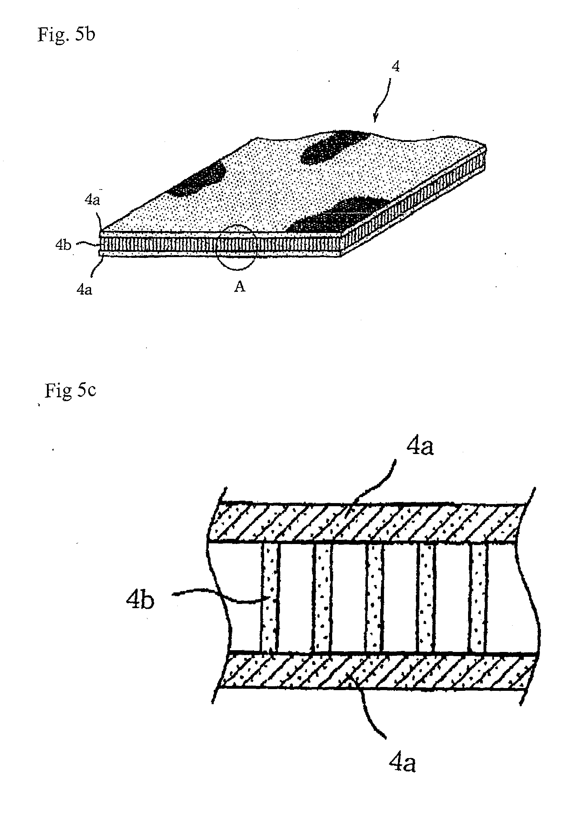

Referring now to Figs. 4 and 5, the heartwood 4 is intervened between the endoderm 2 and the integument 3, as shown in Fig. 5a ― Fig, 5c. The heartwood 4 includes woven fabrics, cotton, glass fiber, synthetic fiber woven fabrics or a sandwiched fabric having two surfaces 4a and a plurality of split fabrics 4b for connecting/supporting the two surfaces, as a basic material. Fig. 5c is an expanded drawing of a portion 'A' in Fig 5b. The cut heartwood 4 is immersed into a solidification solution 7 such as polyurethane resin for wet solidification type (1 liquid phase) or gypsum liquid and is then applied to the endoderm 2 and the integument 3, as shown in Fig. 4c. The heartwood 4 shown in Fig. 5 is intervened between the endoderm 2 and the integument 3, as shown in Fig. 4d, so that the outer circumferences 12 of the endoderm 2 and the integument 3 are integrally adhered. The outer circumference 12 is thermally melted or seamed by a common ultrasonic wave processing or adhered by a tape.

The bent-up splint 100 formed as above has the endoderm 2, the integument 3 and the heartwood 4 integrally formed. The bent-up splint is vacuum-packaged in order for the solidification solution 7 not to dry.

Referring to Fig. 4a ∼ Fig. 4d, there are sequential processes of manufacturing the bent-up splint according to one embodiment of the present invention.

Referring to Fig. 4a, woven fabrics or cotton, sponge, glass fiber or synthetic fiber textiles being materials for the endoderm 2 and the integument 3, are cut within a mold to have a rectangular o a given angle having a horizontal section 1a and a vertical section 1b using a mold along with the shape of the operation by means of an ultrasonic wave processing or a mold cutter. There are shown two sheets of the bent-up plate 5 that is cut by a mold cutter.

Referring to Fig. 4b, the seamed portions of the two sheets of the bent-up plate 5 and the outer circumference portion 6 are seamed or adhered by an ultrasonic wave processing. If they are unfolded, the cross section of the bent-up splint is a 'U' shape. The splint totally has a 'L' shape.

By reference to Fig. 4c, the heartwood 4 is made of a sandwiched fabric having two surfaces 4a and a plurality of split fabrics 4b for connecting/supporting the two surfaces, as a basic material. The heartwood 4 may be formed of sandwiched textiles or woven fabric, cotton, sponge, glass fiber and synthetic fiber textiles is made by the same method to the endoderm and the integument. The heartwood 4 include two sheets of bent-up plates 5' having a rectangular angle or a given angle having a horizontal sections 1a' and a vertical section lb'. A seaming portion 6' of the two sheets of the bent-up plates are seamed or thermally melt by an ultrasonic wave processing. Thus, if they are unfolded, the cross section of them have a 'U' shape and the entire shape has a 'L' shape. The heartwood 7 is immersed into a solidification solution 7 such as gypsum resin or polyurethane resion.

Referring now to Fig. 4d, the heartwood 4 to which the solidification solution 7 is applied is inserted between the endoderm 2 and the integument 3. The heartwood 4 is then seamed to the endoderm 2 and the integument 3 or is adhered by an ultrasonic wave processing, thus forming a bent-up splint 100 as in Fig. 1.

As above, the bent-up splint 100 of the present invention includes the endoderm 2, the integument 3 and the heartwood 4 to which encounter sections 6,6' of the two sheets of the bent-up plates 5,5' having a rectangular angle or a given angle are attached.

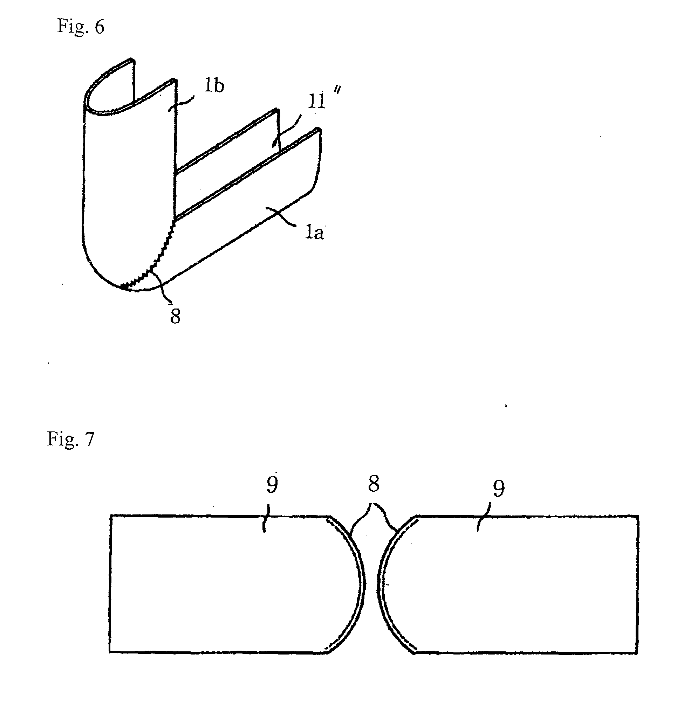

Fig. 6 and Fig. 7 show process of manufacturing the endoderm 2, the integument 3 and the heartwood 4 as bent-up plates according to another embodiment of the present invention. Materials for the endoderm 2, the integument 3 and the heartwood 4 are cut within a mold by means of a mold cutter or an ultrasonic wave process, so that two sheets of plates 9 one ends of which have arc shapes 8 are formed. Then, the central portion of the arc shape 8 is bent and the arc shape 10 are seamed, forming the endoderm 2, the integument 3 and the heartwood 4. The endoderm 2, the integument 3 and the heartwood 4 having the cross sections of U shape 11' as lines 'B-B' in Fig. 1 can be produced.

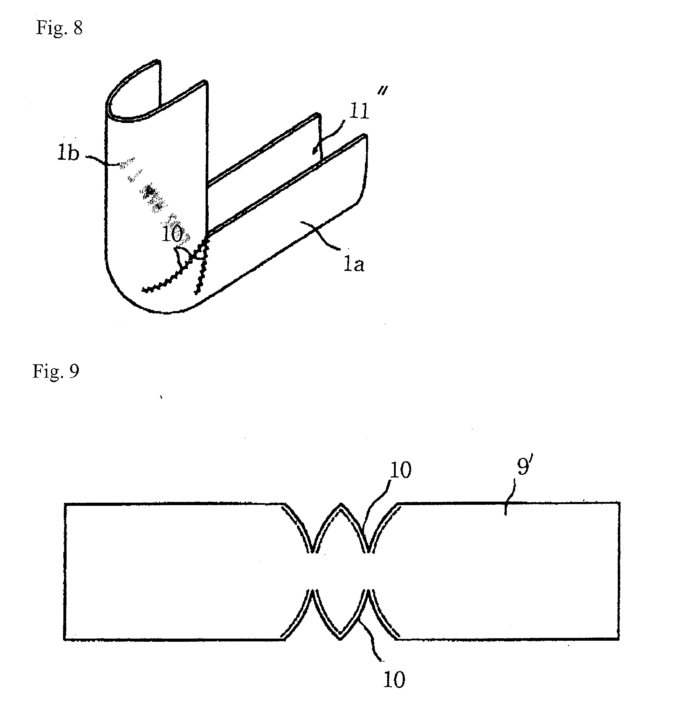

Fig. 8 and Fig. 9 show process of manufacturing the endoderm 2, the integument 3 and the heartwood 4 as bent-up plates according to still another embodiment of the present invention. Materials for the endoderm 2, the integument 3 and the heartwood 4 are cut within a mold by means of a mold cutter or an ultrasonic wave process, so that one sheet of plate 9'. The plate 9' is cut to have lacking sections 10' having a 'W' shape at its both ends. The central portion of the plate 9' is bent and the lacking sections 10' are seamed, forming the endoderm 2, the integument 3 and the heartwood 4. The endoderm 2, the integument 3 and the heartwood 4 having the cross sections of U shape 11' as lines 'B-B' in Fig. 1 can be produced.

The bent-up splint 100 includes the heartwood 4 being a fabric such as Fig. 5a - Fig. 5c applied between the endoderm 2 and the integument 3 by means of solidification solution 7 such as polyurethane resin or gypsum solution.

The shape and angle of the bent-up splint 100 is bent-up in advance similar to the operation state. The heartwood includes a plurality of the split fabrics for fixing the surface fabrics at upper and lower sides. A plurality of through-holes are formed between the split fabrics. Therefore, when the bent-up splint 100 is shaped in line with the shape of the affected parts, the through-holes formed between the splint fabrics of the heartwood has a resilient force, so that it can itself receive variations in the curvature along the shape of the affected parts.

Therefore, there are no any wrinkles on the endoderm 2 and the integument 3 integrally formed with the heartwood, as well as on the surface of the heartwood 4.

In addition, the operation is simple since the range of controlling the range is narrow using the bent-up splint. Further, the affected parts can be closed to the bent-up splint so that the wrinkles such as conventional ones are not formed.

If the bent-up splint 100 is applied to the affected parts and surrounds/fixes the integument 3 of the bent-up splint 100, the heartwood 4 is firmly solidified by the solidification solution 7 in 3 ― 4 minutes, so that the fractured parts do not move.

As mentioned above, the bent-up splint 100 of the present invention is a splint in advance shaped similar to human's curved parts such as elbow, knee and heel. Thus, the splint can be closely adhered to the fractured parts or the affect parts since there are no wrinkles in the bent-up portions. Therefore, the present invention has outstanding advantages that it can reduce the curing time, increase the curing effect, and omit a process of bending up the bent-up portion.

The present invention has been described with reference to a particular embodiment in connection with a particular application. Those having ordinary skill in the art and access to the teachings of the present invention will recognize additional modifications and applications within the scope thereof.

It is therefore intended by the appended claims to cover any and all such applications, modifications, and embodiments within the scope of the present invention.

## Claims

1. A bent-up splint 100 for protecting/supporting the affected parts, comprising:
endoderm 2 and integument 3 the cross section of which are a 'U' 1 1 and each having bent-up sections having a rectangular angle or a given angle, said endoderm and integument are formed by adhering encounter sections 6 of two bent-up plats having a rectangular or a given angle having a horizontal section 1a and a vertical section 1b; and
a heartwood 4 having sandwich textiles having a plurality of fabrics between two fabric surfaces and formed by adhering encounter sections 6' of two bent-up plats having a rectangular or a given angle having a horizontal section 1a' and a vertical section 1b';
wherein the cross section of the heartwood is a 'U' shape, the central portion of the heartwood has a bent-up portion having a rectangular angle or a given angle, and internal and outer surfaces of the heartwood are applied with polyurethane for wet dry or a solidification solution 7,
wherein the heartwood 4 is inserted between the endoderm 2 and an integument 3 and the encounter section 12 in the endoderm 2 and integument 3 are adhered.

2. The bent-up splint as claimed in claim 1, wherein the endoderm 2 and the integument 3 are formed of one of woven fabrics, cotton and sponge, and the heartwood 4 is formed of one of gypsum bandage, woven fabrics, glass fiber, synthetic fiber woven fabrics and sandwiched fabrics.

3. The bent-up splint as claimed in claim 1, wherein the adhering means is seamed or thermally molt by means of an ultrasonic wave processing.

4. The bent-up splint as claimed in claim 1, wherein the bent-up plates include two sheets of plates 9 one end of which have arc sections 8, have a 'U' shape by adhering the arc section 8 and also have a bent-up portion having a given angle.

5. The bent-up splint as claimed in claim 1, wherein said bent-up plate includes a lacking portion 10 of a 'W' shape at both ends at the central portion of one sheet of a plate body 9, has a 'U' shape by means of adhesion of the lacking portion and an bent-up portion having an angle.

6. A method of manufacturing a splint 100 for wrapping/supporting the affected parts, comprising the steps of:
cutting two sheets of bent-up plates to have a rectangular angle or a given angle having a horizontal section 1a and a vertical section 1b along the shape of an operation portion by means of a mold cutter or an ultrasonic wave processing;
adhering encounter section portions 6 of the two bent-up plates having a rectangular angle o a given angle including the horizontal section 1a and the vertical section 1b to produce an endoderm 2 and an integument 3 each having the cross section of a 'U' shape;
adhering encounter sections 6' of the two sheets of bent-up plates made of sandwich fabrics in which a plurality of split fabrics are formed between the two fabric surfaces 4a and having a rectangular angle or a given angle including a horizontal section 1a and a vertical section 1b to form a heartwood 4 the cross section of which is a 'U' shape 11';
immersing the heartwood 4 into a solidification solution 7 made of polyurethane resin or gypsum solution to apply the endoderm 2 and the integument 3 with the solidification solution 7; and
inserting the heartwood 4 between the endoderm 2 and the integument 3 and adhering the outer circumference portion by means of an adhering means.

7. The method as claimed in claim 6, wherein the endoderm 2 and the integument 3 are formed of one of woven fabrics, cotton and sponge, and the heartwood is formed of one of gypsum bandage, woven fabrics, glass fiber, synthetic fiber woven fabrics and sandwiched fabrics.

8. The method as claimed in claim 6, wherein the two sheets of the bent-up plates 5, 5' as the adhering means are overlapped and then seamed or thermally-melt by an ultrasonic wave process so that a seamed portion can be formed in the outer circumference portion 6.

9. The method as claimed in claim 7, wherein one end of the bent-up plates are two sheets of plate bodies 9 of an arc section 8, wherein the cross section of the bent-up plates has a 'U' shape by joining the arc section 8, and has a bent-up portion having an angle.

10. The method as claimed in claim 7, wherein the bent-up plate has a lacking portion 10 of a 'W' shape on both ends at the central portion of one sheet of a plate body 9, has a 'U' shape by means of joining of the lacking portion and has a bent-up portion having an angle.
